(19) Europäisches Patentamt
European Patent Office
Office européen des brevets

(11) Numéro de publication : **0 514 275 A1**

(12) **DEMANDE DE BREVET EUROPEEN**

(21) Numéro de dépôt : **92401331.1**

(22) Date de dépôt : **15.05.92**

(51) Int. Cl.[5] : **C07D 495/04,** A61K 31/40, // (C07D495/04, 335:00, 209:00)

(30) Priorité : **17.05.91 FR 9106037**

(43) Date de publication de la demande : **19.11.92 Bulletin 92/47**

(84) Etats contractants désignés : **PT**

(71) Demandeur : **RHONE-POULENC RORER SA**
**20, avenue Raymond Aron**
**F-92160 Antony (FR)**

(72) Inventeur : **Achard, Daniel**
**26 rue Adrien Tessier**
**F-94320 Thiais (FR)**
Inventeur : **Moutonnier, Claude**
**3 rue Auguste Rodin**
**F-92350 Le Plessis Robinson (FR)**
Inventeur : **Peyronel, Jean-François**
**6 Parc d'Ardenay**
**F-91120 Palaiseau (FR)**
Inventeur : **Tabart, Michel**
**Tour Athènes, 75 rue du Javelot**
**F-75013 Paris (FR)**
Inventeur : **Truchon, Alain**
**73 rue Henri Gorjus**
**F-69004 Lyon (FR)**

(74) Mandataire : **Savina, Jacques et al**
**Rhône-Poulenc Rorer S.A. Direction Brevets (t144) 20 avenue Raymond Aron**
**F-92165 Antony Cédex (FR)**

(54) **Nouveaux dérivés du thiopyranipyrrole, leur préparation et les compositions pharmaceutiques qui les contiennent.**

(57) Nouveaux dérivés de thiopyranopyrrole de formule générale (I) dans laquelle X est un atome d'oxygène ou un radical NH, $R_1$ est phényle éventuellement substitué, cyclohexadiényle, naphtyle, ou hétérocyclyle, $R_2$ est H, halogène, OH, alcoyle, aminoalcoyle, alcoylaminoalcoyle, dialcoytaminoalcoyle, alcoyloxy, alcoylthio, acyloxy, carboxy, alcoyloxycarbonyle éventuellement substitué, benzyloxycarbonyle, amino ou acylamino, et n est 0, 1 ou 2, sous leurs formes stéréoisomères et leurs mélanges, évenluellement leurs sels lorsqu'ils existent et leur préparation.

Les nouveaux dérivés selon l'invention sont particulièrement intéressants comme antagonistes de la substance P.

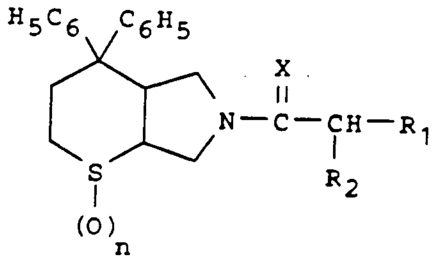

(I)

EP 0 514 275 A1

Jouve, 18, rue Saint-Denis, 75001 PARIS

La présente invention concerne de nouveaux dérivés de thiopyranopyrrole de formule générale :

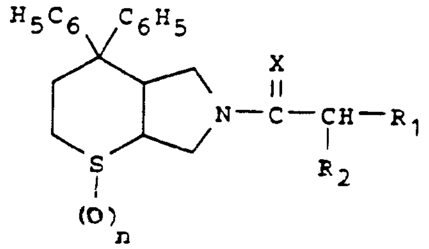

(I)

ainsi que leurs sels lorsqu'ils existent, qui antagonisent les effets de la substance P et sont de ce fait particulièrement intéressants dans les domaines thérapeutiques où cette substance est connue pour intervenir.

Dans le brevet américain 4 042 707 avaient été décrits des produits dérivés de l'isoindole de formule générale:

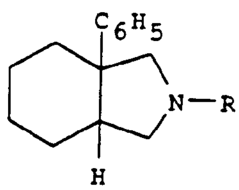

ayant une activité opiacée.

Ces produits n'ont pas d'activité vis à vis de la substance P.

Dans la demande européenne 0068822 ont été décrits des herbicides répondant à la formule générale :

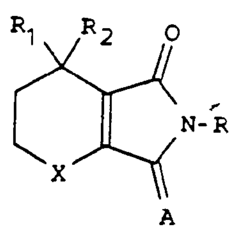

dans laquelle X peut être un atome de soufre, $R_1$ et $R_2$ sont hydrogène ou alcoyle et R est phényle substitué.

Jusqu'à présent, malgré les recherches mises en oeuvre et malgré l'intérêt suscité [M.R. Hanley, TINS,(5) 139 (1982)], il n'avait pratiquement pas été découvert de produit agissant spécifiquement sur la substance P et ayant une structure non peptidique, c'est pourquoi les dérivés de thiopyranopyrrole de formule générale (I) présentent un intérêt considérable.

Dans la formule générale (I) :

- le symbole X représente un atome d'oxygène ou un radical NH,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy ou hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycar-bonyle, amino, acylamino ou alcoyloxycarbonylamino et

- n est un nombre entier de 0 à 2.

Il est entendu que les radicaux alcoyle ou acyle cités ci-dessus contiennent 1 à 4 atomes de carbone en chaîne droite ou ramifiée.

Lorsque $R_1$ contient un atome d'halogène, ce dernier peut être choisi parmi le chlore, le brome, le fluor ou l'iode.

Lorsque $R_1$ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle.

Lorsque $R_1$ représente phényle substitué par une chaîne portant un hétérocycle, ce dernier peut être choisi parmi pyrrolidinyle, morpholino, pipéridinyle, tétrahydropyridinyle, pipérazinyle ou thiomorpholino.

Par ailleurs, les produits de formule générale (I) présentent différentes formes stéréoisomères, il est entendu que les dérivés de thiopyranopyrrole de forme (4aR,7aR) ou de forme (4aS,7aS) à l'état pur, ou sous forme de mélange des formes cis (4aRS,7aRS), entrent dans le cadre de la présente invention.

Les produits de formule générale (I) pour lesquels n=1 présentent également des stéréoisomères axiaux ou équatoriaux au niveau du S-oxyde. Il est entendu que les dérivés R et S en position -1 ainsi que leurs mélanges entrent aussi dans le cadre de la présente invention.

De plus, lorsque le symbole $R_2$ est autre que l'atome d'hydrogène, la chaîne substituée sur le thiopyranopyrrole présente un centre chiral, il est entendu que les formes stéréoisomères et leurs mélanges entrent aussi dans le cadre de la présente invention.

Selon l'invention les dérivés de thiopyranopyrrole de formule générale (I) peuvent être obtenus par action de l'acide de formule générale :

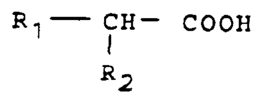

(II)

ou d'un dérivé réactif de cet acide, dans lequel $R_1$ et $R_2$ sont définis comme précédemment, sur un dérivé de thiopyranopyrrole de formule générale :

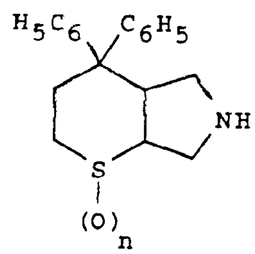

(III)

dans laquelle le symbole n est défini comme précédemment, suivie le cas échéant de la transformation de l'amide obtenu en une amidine pour laquelle X représente un radical NH.

Il est entendu que, les radicaux amino, alcoylamino ou carboxy contenus dans $R_1$ et/ou $R_2$ sont de préférence préalablement protégés.

La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

A titre d'exemple,

- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupemennts acides peuvent être protégés par des radicaux méthyle, éthyle, t.butyle, benzyle, benzyle substitué ou benzhydryle.

De plus, lorsque $R_2$ représente un radical hydroxy, il est préférable de protéger préalablement ce radical. La protection s'effectue par exemple par un radical acétoxy, trialcoylsilyle, benzyle, ou sous forme d'un carbonate par un radical -COORa dans lequel Ra est un radical alcoyle ou benzyle.

Lorsque l'on effectue la condensation d'un dérivé réactif de l'acide de formule générale (II), on opère avantageusement au moyen du chlorure d'acide, de l'anhydride, d'un anhydride mixte ou d'un ester réactif dans lequel le reste de l'ester est par exemple un radical succinimido, benzotriazolyl-1, nitro-4 phényle, dinitro-2,4 phényle, pentachlorophényle ou phtalimido ou un dérivé.

La réaction s'effectue généralement à une température comprise entre -40 et +40°C, dans un solvant organique tel qu'un solvant chloré (dichlorométhane, dichloréthane, chloroforme par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le NN'-carbonyldiimidazole ou l'éthoxy-2 éthoxycarbonyl-1 dihydro-1,2 quinoléine ou bien en milieu hydroorganique, en présence d'un agent alcalin de condensation comme le bicarbonate de sodium, puis on transforme le cas échéant l'amide obtenu en une amidine telle que définie précédemment.

La transformation de l'amide de formule générale (I) en une amidine pour laquelle X est un radical NH s'effectue en préparant le dérivé de formule générale :

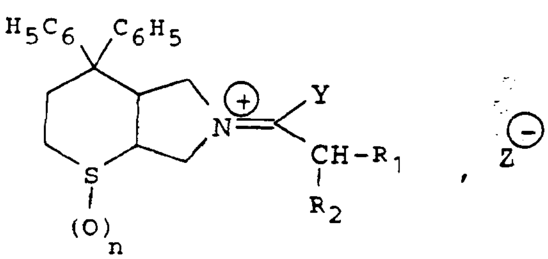 (IV)

dans laquelle $R_1$, $R_2$ et n sont définis comme précédemment, Y représente un atome de chlore, un radical méthoxy ou éthoxy et $Z^-$ représente un ion chlorure, tétrafluoroborate, fluorosulfonate, trifluorométhylsulfonate, méthylsulfate, ou éthylsulfate, puis en faisant agir l'ammoniac sur le dérivé de formule générale (IV).

La préparation du dérivé de formule générale (IV) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La réaction s'effectue dans un solvant chloré (dichlorométhane, dichloréthane par exemple) ou dans un hydrocarbure aromatique (toluène par exemple), à une température comprise entre 0°C et la température de reflux du mélange réactionnel. L'action de l'ammmoniac sur le dérivé de formule générale (IV) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -20°C et la température de reflux du mélange réactionnel.

Il n'est pas indispensable d'avoir isolé le dérivé de formule générale (IV) pour le mettre en oeuvre dans cette réaction.

Selon l'invention les dérivés de thiopyranopyrrole de formule générale (I) pour lesquels le symbole $R_1$ est un radical alcoyloxyphényle dont la partie alcoyle est substituée ou non et le symbole $R_2$ est autre qu'un atome d'halogène ou radical hydroxy peuvent également être préparés à partir d'un dérivé de thiopyranopyrrole de formule générale (I) pour lequel $R_1$ est un radical hydroxyphényle, par action en milieu basique du dérivé halogéné correspondant, de formule générale :

$$Hal - R_4 \quad (V)$$

dans laquelle Hal est un atome d'halogène et $R_4$ est un radical alcoyle éventuellement substitué [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées un hétérocycle tel que défini précédemment dans la formule générale (I)].

La réaction s'effectue généralement en présence d'une base telle qu'un hydrure, un hydroxyde, un alcoolate, ou un carbonate de métal alcalin, dans un solvant organique tel qu'un amide (diméthylformamide par exemple), un hydrocarbure aromatique (toluène par exemple), une cétone (butanone par exemple) ou dans un mélange de ces solvants, à une température comprise entre 20°C et la température de reflux du mélange réactionnel.

Lorsque le radical $R_4$ porte un substituant pouvant interférer avec la réaction, il est entendu que ce dernier est préalablement protégé. La protection et l'élimination du radical protecteur s'effectuent selon les méthodes décrites précédemment.

Selon l'invention, les dérivés de thiopyranopyrrole de formule générale (I) pour lesquels X est un radical NH peuvent également être obtenus à partir du dérivé du thiopyranopyrrole de formule générale (III), par action d'un produit de formule générale :

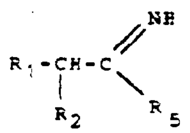

(VI)

éventuellement à l'état de sel, dans laquelle $R_1$ et $R_2$ sont définis comme précédemment et $R_5$ représente un radical alcoyloxy contenant 1 à 4 atomes de carbone en chaîne droite ou ramifiée ou un radical méthylthio, éthylthio, benzylthio ou alcoyloxycarbonylméthylthio.

La réaction est mise en oeuvre au moyen du dérivé de formule générale (VI) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l'acétonitrile à une température comprise entre 0°C et la température de reflux du mélange réactionnel.

Il est entendu que, dans l'éventualité où les radicaux $R_1$ et /ou $R_2$ du produit de formule générale (VI) portent des substituants pouvant interférer avec la réaction, ces derniers doivent être préalablement protégés.

Selon l'invention, les dérivés de thiopyranopyrrole de formule générale (I) pour lesquels n égale 1 ou 2 peuvent également être obtenus par oxydation du dérivé correspondant de formule générale (I) pour lequel n=0.

L'oxydation s'effectue selon les méthodes connues. Par exemple, on opère par action d'un peracide organique (acide percarboxylique ou persulfonique, notamment l'acide perbenzoïque, l'acide chloro-3 perbenzoïque, l'acide nitro-4 perbenzoïque, l'acide peracétique, l'acide pertrifluoracétique, l'acide performique, l'acide permaléique, l'acide monoperphtalique, l'acide percamphorique ou pertoluènesulfonique) ou les peracides minéraux (par exemple l'acide periodique ou persulfurique) . La réaction s'effectue avantageusement dans un solvant chloré (chlorure de méthylène) à une température comprise entre 0 et 25°C. Il est également possible d'opérer au moyen de tertiobutylhydroperoxyde en présence de tetra-isopropylate de titane.

Lorsque l'on veut obtenir un produit de formule générale (I) pour lequel n=2, on opère au moyen de 2 équivalents d'agent oxydant.

Les acides de formule générale (II) peuvent être préparés selon les méthodes décrites ci-après dans les exemples, ou par analogie avec ces méthodes.

Le dérivé de thiopyranopyrrole de formule générale (III) pour lequel n=0 peut être obtenu à partir du dérivé correspondant de formule générale :

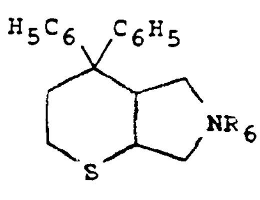

(VII)

dans laquelle $R_5$ représente un radical allyle ou un radical de structure $-CR_aR_bR_c$ dans laquelle $R_a$ et $R_b$ sont des atomes d'hydrogène ou des radicaux phényle éventuellement substitués (par un atome d'halogène, un radical alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non et les radicaux alcoyle contenant 1 à 4 atomes de carbone en chaîne droite ou ramifiée, par élimination du radical $R_5$, par toute méthode connue qui n'affecte pas le reste de la molécule.

Notamment lorsque $R_6$ est autre qu'un radical allyle, le groupement $R_6$ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque $R_6$ est un radical benzhydryle ou trityle, l'élimination peut être effectuée par traitement en milieu acide, en opérant à une température comprise entre 0°C et la température de reflux du mélange réactionnel, dans un alcool, dans un éther, dans l'eau ou directement dans l'acide acétique, l'acide formique ou l'acide trifluoroacétique.

Le groupement $R_6$ peut être également éliminé en faisant agir le chloroformiate de vinyle, le chloroformiate de chloro-1 éthyle ou le chloroformiate de phényle, en passant intermédiairement par un produit de formule générale :

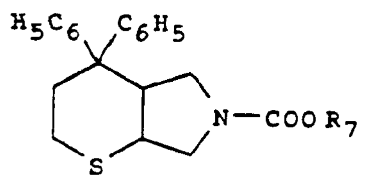

(VIII)

dans laquelle $R_7$ est un radical vinyle, chloro-1 éthyle ou phényle, puis par élimination du radical $R_7$ par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en opérant à une température comprise entre 20°C et la température de reflux du mélange réactionnel.

L'élimination du radical $R_7$ est effectuée par traitement en milieu acide par exemple par l'acide trifluoroacétique, formique, méthanesulfonique, p.toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux $R_7$ citées précédemment, le dérivé de thiopyranopyrrole de formule générale (III) est obtenu à l'état de sel de l'acide employé qui peut être mis en oeuvre directement dans l'étape ultérieure.

Le dérivé de thiopyranopyrrole de formule générale (VII) peut être obtenu par réaction de cycloaddition par action d'un dérivé silylé de formule générale :

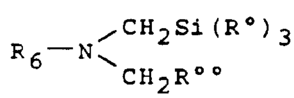

(IX)

dans laquelle $R_6$ est défini comme précédemment, $(R°)_3$ représente des radicaux alcoyle ou des radicaux alcoyle et phényle et $R°°$ représente un radical alcoyloxy, cyano ou phénylthio, sur la déhydrothiapyranone-4 de formule :

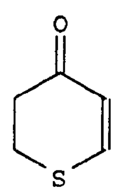

(X)

suivie du traitement du produit obtenu de formule générale :

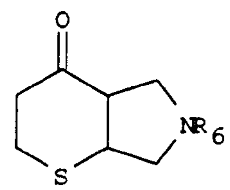

(XI)

successivement par le bromure de phénylmagnésium, puis par le benzène en présence de tétrachlorure de zirconium.

La réaction de cycloaddition s'effectue en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre 0°C et la température de reflux du mélange réactionnel.

La mise en place des radicaux phényle s'effectue selon la méthode décrite plus en détails dans l'exemple 1.

Le dérivé silylé de formule générale (IX) peut être obtenu selon les méthodes décrites par :

- Y. Terao et coll., Chem. Pharm. Bull., 33, 2762 (1985);
- A. Hosomi et coll., Chem. Lett., 1117 (1984) ;
- A. Padwa et coll., Chem. Ber., 119, 813 (1986) ou
- Tetrahedron, 41, 3529 (1985).

Le dérivé du thiopyranopyrrole de formule générale (III) pour lequel n=1 ou 2 peut être obtenu par oxydation du dérivé correspondant de formule générale (III) pour lequel n=0 dont la fonction amine a été préalablement protégée, suivie de l'élimination du radical protecteur.

La réaction s'effectue comme décrit précédemment pour la préparation des dérivés du thiopyranopyrrole de formule générale (I) pour lesquels n=1 ou 2 à partir du dérivé correspondant pour lequel n=0. La mise en place et l'élimination du radical protecteur s'effectuent selon les méthodes habituelles qui n'affectent pas le reste de la molécule; notamment selon les méthodes mentionnées précédemment. Il est également avantageux d'opérer à partir du produit de formule générale (III) pour lequel n=0 sous forme de sel avec un acide minéral (chlorhydrate, sulfate par exemple). Dans la pratique, il est entendu que pour préparer un produit de formule générale (III) pour lequel n=1 ou 2, on opèrera avantageusement à partir d'un dérivé de thiopyranopyrrole de formule générale (VII) ou de formule générale (VIII) obtenu à l'étape précédente, la réaction pouvant être mise en oeuvre directement sur l'un de ces dérivés protégés du produit de formule générale (III).

Les produits de formule générale (III) pour lesquels n=2 peuvent également être obtenus à partir de dihydro-3,4 diphényl-4,4 dioxyde-1,1 2H-thiapyranne de formule :

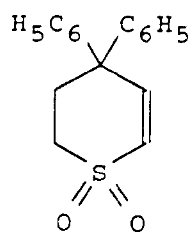

(XII)

par réaction de cycloaddition avec un produit de formule générale (IX), dans des conditions identiques à celles décrites précédemment pour la réaction de cycloaddition de ce produit sur la déhydrothiapyranone-4 de formule (X), suivie de l'élimination du radical protecteur $R_6$ dans les conditions décrites précédemment.

Le dihydro-3,4 diphényl-4,4 dioxyde-1,1 2H-thiapyranne de formule (XII) peut être obtenu par oxydation successive du dihydro-3,4 diphényl-4,4 2H-thiapyranne et du dihydro-3,4 diphényl-4,4-oxyde-1 2H-thiapyranne de formules :

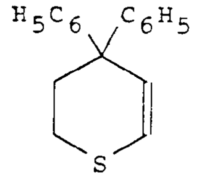

(XIII)

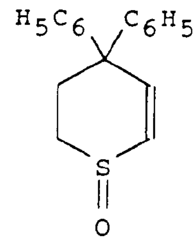

(XIV)

La réaction d'oxydation s'effectue dans les conditions décrites précédemment pour la préparation des produits de formule générale (I). Il n'est pas indispensable d'isoler le S-oxyde de formule générale (XIV) pour l'oxyder en sulfone.

Le dihydro-3,4 diphényl-4,4 2H-thiapyranne de formule générale (XIII) peut être préparé selon ou par analogie avec la méthode décrite ci-après à l'exemple 12.

Il est entendu que les dérivés de thiopyranopyrrole de formule générale (III), (VII) et (VIII) présentent plusieurs formes stéréoisomères. Lorsque l'on veut obtenir un produit de formule générale (I) de stéréoisomérie (4aR,7aR) ou (4aS,7aS), la séparation des formes isomères est mise en oeuvre de préférence au niveau du dérivé de formule générale (III); elle peut également être mise en oeuvre à partir du thiopyranopyrrole de formule générale (I). De même lorsque l'on prépare un dérivé du thiopyranopyrrole pour lequel n=1, la séparation des isomères axial et équatorial s'effectue de préférence au niveau du produit de formule generale (III); elle peut également être mise en oeuvre à partir du thiopyranopyrrole de formule générale (I).

La séparation des stéréoisomères s'effectue selon toute méthode connue et compatible avec la molécule. A titre d'exemple, la séparation peut être effectuée par préparation d'un sel optiquement actif, par action de l'acide L(+) ou D(-) mandélique, ou de l'acide dibenzoyltartrique, puis séparation des isomères par cristallisation. L'isomère recherché est libéré de son sel en milieu basique. La séparation des isomères axial et équatorial peut être effectuée par chromatographie ou cristallisation.

Les nouveaux dérivés de thiopyranopyrrole de formule générale (I) peuvent être purifiés le cas échéant par des méthodes physiques telles que la cristallisation ou la chromatographie.

Le cas échéant, les nouveaux dérivés de formule générale (I) pour lesquels les symboles $R_1$ et/ou $R_2$ contiennent des substituants amino ou alcoylamino et/ou X représente un radical NH, peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de thiopyranopyrrole de formule générale (I) peuvent aussi, le cas échéant, lorsque $R_2$ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-b-phényléthylamine, NN'-dibenzyléthylènediamine, diphénylènediamine, benzhydrylamine, quinine, choline, arginine, lysine, leucine, dibenzylamine).

Les nouveaux dérivés de thiopyranopyrrole selon la présente invention qui antagonisent les effets de la substance P peuvent trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 10 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés se sont montrés actifs à des doses comprises

entre 20 et 2000 nM.

La substance P est connue pour être impliquée dans un certain nombre de domaines pathologiques :

- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiocascular Pharmacology, 10 (suppl. 12), 5172 (1987).

Il a notamment été mis en évidence, par l'étude de plusieurs produits que les nouveaux dérivés de thiopyranopyrrole manifestent une activité analgésique dans la technique de Siegmund E. et coll., Proc. Soc. Exp. Biol. Med., 95, 729 (1957).

| Exemple n° | $DE_{50}$ mg/kg p.o. |
|---|---|
| Exemple 7 | 3 |
| Exemple 10 | 1,7 |

L'étude de plusieurs dérivés de thiopyranopyrrole de formule générale (I) dans la technique de A. Saria et coll., Arch. Pharmacol.,324, 212-218 (1983) adaptée à la souris a permis de mettre en évidence un effet inhibiteur de l'augmentation de la perméabilité capillaire entraîné par le septide (agoniste de la substance P, ce qui témoigne d'une activité anti-inflammatoire :

| Produit de l'exemple n° | $DE_{50}$ mg/kg s.c. |
|---|---|
| Exemple 8 | 0,05 |
| Exemple 10 | < 0,1 |

L'injection de substance P chez l'animal provoque une hypotension. Les produits étudiés dans la technique de C.A. Maggi et coll., J. Auton. Pharmac., 7, 11-32 (1987) manifestent un effet antagoniste chez le rat vis-à-vis de cette hypotension. Notamment les produits administrés à la dose de 1 mg/kg i.v./mn, pendant 5 mn, provoquent un antagonisme de l'hypotension induite par une injection i.v. de 250 mg/kg de substance P.

| Produit de l'exemple n° | % d'inhibition de l'hypotension |
|---|---|
| Exemple 8 | 100 |
| Exemple 10 | 100 |

Par ailleurs, les dérivés de thiopyranopyrrole selon la présente invention ne présentent pas de toxicité, ils se sont montrés atoxiques chez la souris par voie sous cutanée à la dose de 40 mg/kg ou par voie orale à la dose de 100 mg/kg.

D'un intérêt particulier sont les produits suivants :

- {[(diméthylamino-3 propoxy-2) phényl] acétyl}-6 diphényl-4,4 oxyde-1 perhydrothiopyrano,[2,3-c]pyrrole,
- {{[(pyrrolidinyl-1)-3 propoxy-2] phényl} acétyl}-6 diphényl-4,4 oxyde-1 perhydrothiopyrano[2,3-c]pyrrole,
- [(méthoxy-2 phényl)-2 propionyl-(S)]-6 diphényl-4,4 oxyde-1 perhydrothiopyrano[2,3-c]pyrrole,
- {[(diméthylamino-3 propoxy)-2 phényl]-2 acétyl}-6 diphényl-4,4 perhydrothiopyrano[2,3-c]pyrrole.

sous leurs formes stéréoisomères et leur mélanges.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

EXEMPLE 1

A une solution de 1,16 g d'acide diméthylamino-2 phénylacétique dans 20 $cm^3$ de dichlorométhane sec, on ajoute 1,18 g de N,N'-carbonyldiimidazole. On agite 30 minutes à +5°C puis on ajoute une solution de 2,0 g de chlorhydrate de diphényl-4,4 perhydrothiopyrano[2 ,3-c]pyrrole-(4aRS,7aRS) et de 1,83 $cm^3$ de triéthylamine dans 20 $cm^3$ de dichlorométhane. Le mélange réactionnel est agité pendant 1 heure à 20°C puis est lavé 2 fois par 50 $cm^3$ d'eau et séché sur sulfate de magnésium. La solution est filtrée, concentrée à sec sous pression réduite (2,7 kPa). L'huile obtenue est chromatographiée sur une colonne de gel de silice (0,04 mm-0,06 mm, diamètre 2,8 cm, hauteur 26 cm), en éluant sous une pression de 0,6 bar d'azote par un mélange de cyclohexane et d'acétate d'éthyle (60/40 en volumes), en recueillant des fractions de 60 $cm^3$. Les fractions 6 à 20 sont réunies et concentrées à sec sous pression réduite (2,7 kPa), le résidu est cristallisé dans un mélange d'acétonitrile et d'oxyde de diisopropyle. Les cristaux sont essorés, séchés. On obtient 2,16 g de [(diméthylamino-2 phényl)acétyl]-6 diphényl-4,4 perhydrothiopyrano [2,3-c] pyrrole-(4aRS,7aRS) sous la forme d'un solide blanc fondant à 163°C.

Le chlorhydrate de diphéthyl-4,4 perhydrothiopyrano[2,3-c] pyrrole-(4aRS,7aRS) peut être préparé de la façon suivante:

On traite 4,35 g de diphényl-4,4 vinyloxycarbonyl-6 perhydrothiopyrano [2,3-c]pyrrole-(4aRS,7aRS) par 30 $cm^3$ d'une solution 5,7 N d'acide chlorhydrique dans le dioxane sec pendant 30 minutes à 20°C. La solution est concentrée à sec sous pression réduite (2,7 kPa), le résidu est repris par 150 $cm^3$ d'éthanol, la solution résultante est portée au reflux pendant 30 minutes, puis est concentrée à sec. Le solide obtenu est lavé par 50 $cm^3$ d'éther éthylique, essoré, séché. On obtient 3,64 g de chlorhydrate de diphényl-4,4 perhydrothiopyrano[2,3-c]pyrrole-(4aRS,7aRS), sous la forme d'un solide blanc.

Spectre infra-rouge (KBr, bandes caractéristiques, $cm^{-1}$): 3060, 3030, 3000, 2250, 1600, 1495, 1580, 1450, 755, 710, 700.

Spectre RMN du proton (DMSO-$d_6$,signaux principaux): 2,2 à 2,9 (mt, 4H, $CH_2$ en 2 et $CH_2$ en 3); 2,4 et 3,3 (mt, 2H, $CH_2$ en 5); 3,08 (d, J=12,5, 1H, 1H en 7); 3,7 (mt, 1H, H en 4a); 4,16 (t, J=5, 1H, H en 7a); 7,1 à 7,5 (mt, 10 H, aromatiques).

Le diphényl-4,4 vinyloxycarbonyl-6 perhydrothiopyrano[2,3-c]pyrrole-(4aRS,7aRS) peut être préparé de la manière suivante:

A 6,2 g de benzyl-6 diphényl-4,4 perhydrothiopyrano[2,3-c]pyrrole-(4aRS,7aRS), dans 50 $cm^3$ de dichloro-1,2 éthane, on ajoute 1,72 $cm^3$ de chloroformiate de vinyle. Le mélange est porté au reflux pendant 15 minutes puis concentré à sec sous pression réduite (2,7 kPa). Le résidu est chromatographié sur une colonne

de gel de silice (0,04 mm-0,06 mm, diamètre 25 cm), en éluant sous une pression de 0,6 bar d'azote, par un mélange de cyclohexane et d'acétate d'éthyle (90/10 en volumes), en recueillant des fractions de 60 $cm^3$. Les fractions 5 à 16 sont réunies et concentrées à sec sous pression réduite (2,7 kPa), le résidu est trituré dans 70 $cm^3$ d'oxyde de diisopropyle, la suspension est filtrée, le solide essoré et séché. On obtient 4,35 g de diphényl-4,4 vinyloxycarbonyl-6 perhydrothiopyrano[2,3-c] pyrrole-(4aRS,7aRS), sous la forme d'un solide blanc fondant à 160°C.

Le benzyl-6 diphényl-4,4 perhydrothiopyrano[2,3-c]pyrrole-(4aRS,7aRS) peut être préparé de la façon suivante:

A une solution de 12,2 g d'hydroxy-4 phényl-4 benzyl-6 perhydrothiopyrano[2,3-c]pyrrole-(4RS,4aSR,7aRS) dans 180 $cm^3$ de benzène, on ajoute 43,7 g de tétrachlorure de zirconium. Le mélange réactionnel est porté au reflux pendant 1 heure puis amené à 20°C et dilué par 200 $cm^3$ de dichlorométhane. A la solution résultante refroidie, on ajoute 150 $cm^3$ d'une solution aqueuse d'hydroxyde de sodium 4N. La suspension obtenue est filtrée, le filtrat est décanté, la phase organique est lavée par 200 $cm^3$ de solution aqueuse saturée de chlorure de sodium, séchée sur sulfate de magnésium, concentrée à sec sous pression réduite (2,7 kPa). L'huile obtenue est chromatographiée sur une colonne de gel de silice (0,04 mm-0,06 mm, diamètre 5,2 cm, hauteur 39 cm), en éluant sous une pression d'azote de 0,6 bar par un mélange de cyclohexane et d'acétate d'éthyle (90/10 en volumes), en recueillant des fractions de 125 $cm^3$. Les fractions 19 à 32 sont réunies et concentrées à sec sous pression réduite (2,7 kPa). L'huile obtenue est cristallisée dans 200 $cm^3$ d'oxyde de diisopropyle, les cristaux sont essorés, séchés. On obtient 6,2 g de benzyl-6 diphényl-4,4 perhydrothiopyrano[2,3-c] pyrrole-(4aRS,7aRS), sous la forme de cristaux orangés, fondant à 130°C.

L'hydroxy-4 phényl-4 benzyl-6 perhydrothiopyrano[2,3-c]pyrrole-(4RS,4aSR,7aRS) peut être préparé de la façon suivante:

A une solution de bromure de phénylmagnésium préparée à partir de 19,8 $cm^3$ de bromobenzène et de 4,52 g de magnésium sec, dans 120 $cm^3$ d'éther éthylique anhydre, on ajoute en 30 minutes une solution de 21,15 g de benzyl-6 oxo-4 perhydrothiopyrano[2,3-c]pyrrole (4aRS,7aSR) dans 150 $cm^3$ d'éther éthylique anhydre. Le mélange réactionnel est agité à la température du reflux pendant 3 heures, puis pendant 20 heures à 20°C. Le mélange, additionné de 200 $cm^3$ d'éther éthylique est agité avec 600 $cm^3$ de solution aqueuse saturée de chlorure d'ammonium. La phase aqueuse est extraite par 200 $cm^3$ d'éther éthylique, les deux extraits éthérés réunis sont lavés deux fois par 300 $cm^3$ de solution aqueuse saturée de chlorure de sodium puis séchés sur sulfate de magnésium et concentrés à sec sous pression réduite (5,4 kPa) à 35 °C. On obtient. 12,2 g d'hydroxy-4 phényl-4 benzyl-6 perhydrothiopyrano [2,3-c]pyrrole-(4RS, 4aSR, 7aRS), sous la forme d'un solide blanc fondant à 137 °C.

Le benzyl-6 oxo-4 perhydrothiopyrano[2,3-c]pyrrole-(4aRS,7aSR) peut être préparé de la manière suivante:

A une solution de 20 g de déhydrothiapyrannone-4 et de 54 $cm^3$ de N-butoxyméthyl N-triméthylsilylméthyl benzylamine dans 100 $cm^3$ de dichlorométhane anhydre, on ajoute 5 gouttes d'acide trifluoroacétique et agite pendant 4 heures, en maintenant la température à 20°C. Le mélange réactionnel est agité avec 5 g de carbonate de potassium, filtré, puis concentré à sec sous pression réduite (2,7 kPa) . Le résidu huileux est chromatographié sur une colonne de gel de silice (0,04 mm-0,06 mm, diamètre 9, 2 cm), en éluant sous une pression de 0,6 bar d'azote, par un mélange de cyclohexane et d'acétate d'éthyle (90/10 en volumes), puis par le mélange cyclohexane et acétate d'éthyle (75/25 en volumes), en recueillant des fractions de 250 $cm^3$. Les fractions 35 à 56 sont réunies et concentrées à sec sous pression réduite (2,7 kPa). On obtient 24 g de benzyl-6 oxo-4 perhydrothiopyrano [2, 3-c] pyrrole-(4aRS, 7aSR), sous la forme d'une huile jaune.

Spectre infra-rouge (Solution $CCl_4$, bandes caractéristiques, $cm^{-1}$): 3090, 3070, 3025, 2925, 2850, 2800, 2730, 1710, 1600, 1585, 1495, 1475, 1450, 700.

Spectre RMN du proton ($CDCl_3$,signaux principaux): 2,42 (dd, J=10 et 7, 1H, 1H en 7); 2,66 (mt, 2H, $CH_2$ en 5); 3,05 (mt, 1H, H en 4a); 3,1 (dd, J=10 et 7,5, 1H du $CH_2$ en 7); 3,61 (s, 2H, N-$CH_2$-Ar); 3,8 (dt, J=7,5 et 7, 1H, H en 7a); 7,15 à 7,35 (mt, 5H aromatiques).

## EXEMPLE 2

En opérant comme à l'exemple 1 à partir de 1,85 g de bromhydrate d'acide [(pyrrolidinyl-1)-2 phényl]acétique, et de 2,0 g de chlorhydrate diphényl-4,4 perhydrothiopyrano[2,3-c]pyrrole-(4aRS,7aRS), on obtient 0,90 g de {[(pyrrolidinyl-1)-2 phényl]acétyl}-6 diphényl-4,4 perhydro-thiopyrano[2,3-c]pyrrole-(4aRS,7aRS), sous la forme de cristaux blancs fondant à 166°C.

EXEMPLE 3

A une solution refroidie à 0°C de 2,63 g de chlorhydrate de diphényl-4,4 perhydrothiopyrano[2,3-c]pyrrole-(4aRS,7aRS) et de 2,42 $cm^3$ de triéthyl-amine dans 25 $cm^3$ de dichlorométhane, on ajoute en 5 minutes une solution de 1,15 $cm^3$ de chlorure de phénylacétyle dans 25 $cm^3$ de dichlorométhane. Après agitation une heure à 0°C et une heure à 20°C, on ajoute 20 $cm^3$ de dichlorométhane. Le mélange réactionnel est lavé deux fois par 100 $cm^3$ d'eau distillée, séché sur sulfate de magnésium, puis concentré à sec sous pression réduite (2,7 kPa). L'huile obtenue est chromatographiée sur une colonne de gel de silice (0,04-0,06 mm, diamètre 3,5 cm, hauteur 26 cm), en éluant sous une pression d'azote de 0,4 bar par un mélange de cyclohexane et d'acétate d'éthyle (80/20 en volumes), en recueillant des fractions de 125 $cm^3$. Les fractions 19 à 26 sont réunies et concentrées à sec sous pression réduite (2,7 kPa). On obtient 0,87 g de phénylacétyl-6 diphényl-4,4 perhydrothiopyrano [2,3-c]pyrrole-(4aRS,7aRS), sous la forme d'un solide blanc fondant à 210°C.

EXEMPLE 4

A une solution de 0,92 g d'hydroxy-2 phényl acétique dans 30 $cm^3$ de dichlorométhane sec, on ajoute 0,58 $cm^3$ de chloroformiate d'éthyle. Après 15 minutes d'agitation à 20°C, on refroidit le mélange à -15°C et ajoute 0,85 $cm^3$ de triéthylamine. Après 2 heures d'agitation à -15°C, on ajoute en 20 minutes une suspension de 2 g de chlorhydrate de diphényl-4,4 perhydrothiopyrano[2,3-c]pyrrole-(4aRS,7aRS) et de 1,70 $cm^3$ de triéthylamine dans 30 $cm^3$ de dichlorométhane. Après agitation pendant 20 heures à 20°C, le mélange réactionnel est lavé par 50 $cm^3$d'acide chlorhydrique 1N, 50 $cm^3$ de solution aqueuse saturée de chlorure de sodium puis séché sur sulfate de magnésium, et concentré à sec sous pression réduite (2,7 kPa). Le résidu est cristallisé dans 20 $cm^3$ de dichlorométhane, les cristaux sont lavés par de l'oxyde de diisopropyle, essorés et séchés sous pression réduite (2,7 kPa). On obtient 1,02 g de diphényl-4,4 [(hydroxy-2 phényl)acétyl]-6 perhydrothiopyrano[2,3-c] pyrrole-(4aRS,7aRS), sous la forme de cristaux blancs fondant à 248°C.

EXEMPLE 5

A une solution refroidie à 0°C de 1,16 g d'acide méthoxy-2 phénylacétique dans 20 $cm^3$ de dichlorométhane sec, on ajoute 1,13 g de N,N'-carbonyldiimidazole. On agite 40 minutes à 0°C puis on ajoute une solution de 2,15 g de chlorhydrate de diphényl-4,4 perhydrothiopyrano[2,3-c]pyrrole-(4aRS,7aRS) et de 0,9 $cm^3$ de triéthylamine dans 20 $cm^3$ de dichlorométhane. Le mélange réactionnel est agité pendant une heure à 0°C puis est lavé par une solution aqueuse saturée d'hydrogénocarbonate de sodium. La phase organique est séchée sur sulfate de magnésium, concentrée à sec sous pression réduite (2,7 kPa). Le solide obtenu est lavé par 30 $cm^3$ d'éther éthylique, 30 $cm^3$ d'oxyde de diisopropyle, puis est séché sous pression réduite (2,7 kPa). On obtient 2,66 g de diphényl-4,4 [(méthoxy-2 phényl)acétyl]-6 perhydrothiopyrano[2,3-c]pyrrole-(4aRS,7aRS), sous la forme d'un solide blanc fondant à 172°C.

EXEMPLE 6

Le [(méthoxy-2 phényl)-2 propionyl-(S)]-6 diphényl-4,4 perhydrothiopyrano[2,3-c]pyrrole-mélange des formes (4aR,7aR) et (4aS,7aS) peut être préparé en opérant comme décrit dans l'exemple 5, à partir de 0,89 g d'acide (méthoxy-2 phényl)-2 propionique-(S) et de chlorhydrate de diphényl-4,4 perhydrothiopyrano[2,3-c]pyrrole-(4aRS,7aRS). On obtient 1,46 g de [(méthoxy-2 phényl)-2 propionyl-(S)]-6 diphényl-4,4 perhydrothiopyrano[2,3-c]pyrrole mélange des formes (4aR,7aR) et (4aS,7aS) sous la forme d'une meringue blanche.

Première forme :

Spectre infra-rouge (bandes caractéristiques, $cm^{-1}$): 3095, 3055, 3025, 2950, 2930, 2875, 2835, 1630 1595, 1490, 1565, 1415, 1240, 1030, 750, 700.

Spectre RMN du proton (DMSO-$d_6$ + $CF_3COOD$, à température ambiante, on observe le mélange des deux rotamères ; signaux caractéristiques): 1,15 et 1,20 (2d, J=7,5, 3H, $CH_3$); 2,1-2,9 (mt, 5H, 2 $CH_2$ en 5 et 3 + H en 4a); 3,36 et 3,8 (2s, 3H, $OCH_3$); 6,7 à 7,4 (mt, 14H, aromatiques).

Deuxième forme :

Spectre infra-rouge (bandes caractéristiques, $cm^{-1}$): 3095, 3060, 3025, 2960, 2930, 2870, 2835, 1640, 1600, 1495, 1465, 1425, 1240, 1035, 755, 700.

Spectre RMN du proton ($DMSO-d_6$ + $CF_3COOD$, à température ambiante, on observe le mélange des deux rotamères ; signaux caractéristiques): 1,1 et 1,18 (2d, J=7,5, 3H, $CH_3$); 2,1-2,35 (mt, 1H, $CH_2$ en 3); 2,35-3,10 (mt, 3H, $CH_2$ en 5 + H en 4a); 3,6 et 3,8 (2s, 3H, $OCH_3$); 3,95 et 4,02 (mt, 1H, H en 7a); 6,7 à 7,4 (mt, 14H, aromatiques).

L'acide (méthoxy-2 phényl)-2 propionique-(S) peut être obtenu de la manière suivante :

L'acide (méthoxy-2 phényl)-2 propionique-(S) peut être préparé, par analogie avec les méthodes décrites par D.A Evans et coll.,Tetrahedron, 44, 5525,(1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. $[\alpha]_D^{20}$ = +84,6° (c=1 ; $CHCl_3$).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1, 89 g d'hydrure de sodium (dispersion à 80% dans la vaseline) dans 200 cm³ de tétrahydrofuranne sec on ajoute à température ambiante 9, 38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35, 27 cm³ de butyllithium 1,6 M dans l'hexane à une solution refroidie à -78°C de 10 g de méthyl-4 phényl-5 oxazolidinone-2-(4S, 5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle ; aprés décantation la phase organique est lavée deux fois par 100 cm³ d'eau, puis deux fois par 100 cm³ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0, 04-0, 06 mm, diamètre 4,8 cm, hauteur 36 cm), en éluant sous une pression de 0, 6 bar d'azote par un mélange de cyclohexane et d'acétate d'éthyle (85/15 puis 80/20 en volumes) et en recueillant des fractions de 50 cm³. Les fractions 14 à 31 sont réunies et concentrées à sec sous pression réduite (2,7 kPa). On obtient 13,6 g de méthyl-4 phényl-5 (méthoxy-2 phénylacétyl) -3 oxazolidinone-2-(4S, 5S) sous la forme d'une huile jaune.

Exemple 7

En opérant selon le mode opératoire de l'exemple 8 ci-après, à partir de 1,82 g de diphényl-4,4 oxyde-1 perhydrothiopyrano[2,3-c]pyrrole-(1RS,4aRS,7aRS) et de 1,39 g d'acide [(diméthylamino-3 propoxy-2) phényl] acétique, on obtient 0,3 g de {[(diméthylamino-3 propoxy-2) phényl] acétyl}-6 diphényl-4,4 oxyde-1 perhydrothiopyrano [2,3-c]pyrrole-(1RS,4aRS,7aRS) sous la forme de cristaux blancs fondant à 150°C.

Le diphényl-4,4 oxyde-1 perhydrothiopyrano[2,3-c]pyrrole-(1RS,4aRS,7aRS) peut être préparé de la façon suivante:

On traite 3,98 g de diphényl-4,4 oxyde-1 tertiobutyloxycarbonyl-6 perhydrothiopyrano[2,3-c]pyrrole (mélange d'isomères 1RS,4aSR,7aSR et 1RS,4aRS,7aRS) par 40 cm³ d'un mélange d'acide chlorhydrique concentré (37% d'acide chlorhydrique) et de dioxane (1/2 en volumes) pendant 48 heures à 20°C. La solution est concentrée à sec à 40°C sous pression réduite (2,7 kPa). L'huile obtenue est reprise par 30 cm³ de dichlorométhane, la solution est lavée par 60 cm³ de solution aqueuse d'hydroxyde de sodium 2N, la phase aqueuse est extraite par 20 cm³de dichlorométhane. Les extraits organiques sont réunis, séchés sur sulfate de magnésium, concentrés à sec à 40°C sous pression réduite (2,7 kPa). Le résidu est repris par de l'oxyde de diisopropyle, puis est concentré à sec à 40°C sous pression réduite (2,7 puis 0,13 kPa). On obtient 3,0 g de diphényl-4,4 oxyde-1 perhydrothiopyrano[2,3-c]pyrrole(1RS,4aRS,7aRS) sous la forme d'une meringue blanche.

Spectre infra-rouge (KBr, bandes caractéristiques, $cm^{-1}$): 3080, 3055, 3025, 2950, 2920, 2880, 2860, 1595, 1580, 1490, 1440, 1020, 760, 740, 700.

Spectre RMN du proton ($DMSO-d_6$ + $CF_3CCOD$, signaux principaux): 2,26 (t large, J=14, 1H, 1H en 3); 2,42 (dd, J=10 et 9, 1H, $CH_2$ en 5); 2,55 (dd large, J=14 et 4, 1H, 1H en 3); 3,68 (t, J=6, 1H, H en 7a); 3,82 (d, J=14, 1H, H en 7); 3,8 à 4 (mt, 1H, CH en 4a); 4,15 (dd, J=14 et 6, 1H, H en 7);7,1 à 7,5 (mt, 10H aromatiques).

Le diphényl-4,4 oxyde-1 tertiobutyloxycarbonyl-6 perhydrothiopyrano [2,3-c]pyrrole (mélange d'isomères 1RS,4aSR,7aSR et 1RS,4aRS,7aRS) peut être préparé de la façon suivante:

A une solution refroidie à 0°C de 4,2 g de diphényl-4,4 tertiobutyloxycarbonyl-6 perhydrothiopyrano[2,3-c]pyrrole-(4aRS,7aRS) dans 30 $cm^3$ de dichlorométhane sec, on ajoute une solution de 2,3 g d'acide chloro-3 peroxybenzoïque (à 85%) dans 20 $cm^3$ de dichlorométhane. Après 1,5 heures d'agitation à 3°C et 1,5 heures à 20°C, le mélange réactionnel est lavé 2 fois par 100 $cm^3$ de solution aqueuse saturée de bicarbonate de sodium, puis par 100 $cm^3$ d'eau distillée, séché sur sulfate de magnésium, concentré à sec à 35°C sous pression réduite (2,7 kPa). Le résidu est cristallisé dans de l'acétate d'éthyle, les cristaux sont lavés par de l'acétate d'éthyle et de l'oxyde de diisopropyle, essorés, puis séchés sous pression réduite (2,7 kPa). On obtient 3,98 g de diphényl-4,4 oxyde-1 tertiobutyloxycarbonyl-6 perhydrothiopyrano [2,3-c]pyrrole-(mélange d'isomères 1RS,4aSR,7aSR et 1RS,4aRS,7aRS) sous la forme de cristaux blancs utilisés tel quel dans la réaction suivante.

Le diphényl-4,4 tertiobutyloxycarbonyl-6 perhydrothiopyrano[2,3-c] pyrrole-(4aRS,7aRS) peut être préparé de la manière suivante:

A une suspension de 4,0 g de chlorhydrate de diphényl-4,4 perhydrothiopyrano[2,3-c]pyrrole-(4aRS,7aRS) et de 1,70 $cm^3$ de triéthylamine dans 60 $cm^3$ de dichlorométhane sec, on ajoute par fractions de 0,5 g, 2,89 g de dicarbonate de ditert-butyle puis 0,15 g de diméthylamino-4 pyridine. On agite 20 heures à 20°C puis on lave la solution réactionelle par 2 fois 100 $cm^3$ de solution aqueuse d'acide citrique de pH 4, par 100 $cm^3$ d'eau, sèche sur sulfate de magnésium, concentre à sec à 35°C sous pression réduite (2,7 kPa). Le résidu est cristallisé dans de l'éther éthylique, les cristaux sont essorés, et séchés. On obtient 4,27 g de diphényl-4,4 tertiobutyloxycarbonyl-6 perhydrothiopyrano[2,3-c]pyrrole-(4aRS,7aRS) sous la forme de cristaux roses fondant à 162°C.

Le diphényl-4,4 oxyde-1 perhydrothiopyrano[2,3-c]pyrrole-(1RS,4aRS,7aRS) peut être aussi préparé de la façon suivante:

A une solution refroidie à -3°C de 25 g de diphényl-4,4 perhydrothiopyrano[2,3-c]pyrrole-(4aRS,7aRS) dans 500 $cm^3$ de dichlorométhane et 100 $cm^3$ de méthanol, on ajoute en 40 minutes une solution de 15,4 g d'acide chloro-3 peroxybenzoïque (à 85%) dans 400 $cm^3$ de dichlorométhane. Après une heure d'agitation à -3°C, le mélange réactionnel est lavé par 200 $cm^3$ d'une solution aqueuse d'hydrogénocarbonate de potassium à 10% et 100 $cm^3$ de cette même solution, puis séché sur sulfate de magnésium, et concentré à sec à 40°C sous pression réduite (2,7 kPa). Le résidu est cristallisé dans 50 $cm^3$ d'acétate d'éthyle, les cristaux sont repris par 200 $cm^3$ de dichlorométhane, la solution obtenue est lavée par 75 $cm^3$ d'une solution aqueuse d'hydroxyde de sodium 1N puis séchée sur sulfate de magnésium et concentrée à sec. Le résidu est cristallisé dans 30 $cm^3$d'acétate d'éthyle, les cristaux sont lavés par de l'acétate d'éthyle, essorés et séchés. On obtient 13,6 g de diphényl-4,4 oxyde-1 perhydro-thiopyrano[2,3-c]pyrrole(1RS,4aRS,7aRS) sous la forme de cristaux blancs fondant à 174°C.

## EXEMPLE 8

A une solution refroidie à 0°C de 1,06 g de (-)-diphényl-4,4 oxyde-1 perhydrothiopyrano[2,3-c]pyrrole-(1R*, 4aR*, 7aR*) et de 0,81 g d'acide [(diméthylamino-3 propoxy-2)phényl]-2 acétique dans 60 $cm^3$ de dichlorométhane sec, on ajoute 0,03 g d'hydrate d'hydroxybenzotriazole, puis 0,77 g de chlorhydrate de (diméthylamino-3 propyl)-1 éthyl-3 carbodiimide. Après 2 heures d'agitation à 0°C et 20 heures à 20°C, le mélange réactionnel est lavé par 20 $cm^3$ d'eau puis séché sur sulfate de magnésium, et concentré à sec sous pression réduite (2,7 kPa). Le résidu est chromatographié sur colonne de gel de silice (granulométrie 0,04-0,06 mm, diamètre 2,4 cm, hauteur 35 cm) , en éluant sous une pression de 0,6 bar d'azote par un mélange d'acétate d'éthyle, d'acide acétique et d'eau (60/10/10 en volumes) et en recueillant des fractions de 50 $cm^3$. Les fractions 8 à 19 sont réunies et concentrées à sec sous pression réduite (2,7 kPa). Le résidu est repris avec 60 $cm^3$ de dichlorométhane, la solution est lavée par 20 $cm^3$ de solution aqueuse d'hydroxyde de sodium 1N, séchée sur sulfate de magnésium, puis concentrée à sec. Le solide obtenu est recristallisé dans un mélange d'acétate d'éthyle et d'éther éthylique, les cristaux sont lavés par de l'oxyde de diisopropyle, essorés, et séchés. On obtient 0,99 g de (-)-{[(diméthylamino-3 propoxy-2)phényl]-2 acétyl}-6 diphényl-4,4 oxyde-1 perhydrothiopyrano [2,3-c]pyrrole-(1R*,4aR*,7aR*) sous la forme d'un solide blanc fondant à 120°C. $[\alpha]^{20}D$ = -323° (c = 0,5; acide acétique).

Le (-)-diphényl-4,4 oxyde-1 perhydrothiopyrano[2,3-c]pyrrole-(1R*,4aR*,7aR*) peut être préparé de la façon suivante:

A 7,15 g de diphényl-4,4 oxyde-1 perhydrothiopyrano [2,3-c] pyrrole-(1RS,4aRS,7aRS) on ajoute 3,5 g d'acide mandélique-(S) et 90 $cm^3$ d'un mélange d'acétonitrile et d'eau (99/1 en volumes). Après agitation la solution résultante est laissée pendant 48 heures à température ambiante. Les cristaux obtenus sont essorés, lavés par avec le mélange acétonitrile-eau, puis séchés. Les cristaux sont repris par 200 $cm^3$ du mélange acétonitrile-eau bouillant et après filtration à chaud, la solution obtenue est laissée 5 heures à température ambiante. Les cristaux sont essorés, lavés 2 fois par 10 $cm^3$ d'acétonitrile, puis séchés. On obtient 1,5 g de mandélate-(S) de diphényl-4,4 oxyde-1 perhydrothiopyrano[2,3-c]pyrrole-(1R*,4aR*,7aR*) $[a]^{20}D$ = -228°, (c = 0,44; acide acétique). Le filtrat est laissé 20 heures à température ambiante, les cristaux obtenus sont essorés, lavés 2 fois par 5 $cm^3$ d'acétonitrile, puis séchés. On obtient 0,62 g de mandélate-(S) de diphényl-4,4 oxyde-1 perhydrothiopyrano[2,3-c]pyrrole-(1R*,4aR*,7aR*); $[\alpha]^{20}D$ = -230°, (c = 0, 45; acide acétique).

A 2,06 g de mandélate-(S) de diphényl-4,4 oxyde-1 perhydrothiopyrano[2,3-c]pyrrole-(1R*,4aR*,7aR*) on ajoute 40 $cm^3$ de dichlorométhane et 7,0 $cm^3$ de soude aqueuse 1N. Le mélange est agité quelques minutes après dissolution du produit de départ, la phase organique est séchée sur sulfate de magnésium, concentrée à sec sous pression réduite (2,7 kPa). Le résidu est délité dans un mélange d'acétate d'éthyle et d'éther éthylique, le solide est lavé par de l'oxyde de diisopropyle, et séché. On obtient 1,14 g de (-)-diphényl-4,4 oxyde-1 perhydrothiopyrano [2,3-c]pyrrole-(1R*,4aR*,7aR*) sous la forme d'un solide blanc fondant à 192°C. $[\alpha]^{20}D$ = -405° , (c = 0,46; acide acétique).

EXEMPLE 9

A une solution refroidie à +5°C de 0,69 g de diphényl-4,4 oxyde-1 perhydrothiopyrrano[2,3-c]pyrrole-(7RS,4aRS,7aRS) et de 0,68 g d'acide {((pyrrolidinyl-1)-3 propoxy-2]phényl}acétique dans 25 $cm^3$ de dichlorométhane sec, on ajoute 0,03 g d'hydrate d'hydroxybenzotriazole, puis une solution de 0,5 g de chlorhydrate de (diméthylamino-3 propyl)-1 éthyl-3 carbodiimide dans 20 $cm^3$ de dichlorométhane sec. Après 2 heures d'agitation à +5°C et 20 heures à 20°C, le mélange réactionnel est lavé 2 fois par 50 $cm^3$ d'eau distillée et séché sur sulfate de magnésium puis concentré à sec sous pression réduite (2,7 kPa). Le résidu obtenu est chromatographié sur colonne de gel de silice (granulométrie 0,04-0,06 mm, diamètre 2,4 cm, hauteur 32 cm), en éluant sous une pression de 0,8 bar d'azote par un mélange d'acétate d'éthyle, d'acide acétique, et d'eau (80/20/20 en volumes) et en recueillant des fractions de 25 $cm^3$. Les fractions 21 à 50 sont réunies et concentrées à sec sous pression réduite (2,7 kPa). Le résidu est cristallisé dans 8 $cm^3$ d'acétate d'éthyle, les cristaux sont lavés par de l'acétate d'éthyle et de l'oxyde de diisopropyle puis séchés. On obtient 0,45 g de {[(pyrrolidinyl-1)-3 propoxy-2]phényl}acétyl}-6 diphényl-4,4 oxyde-1 perhydrothiopyrano[2,3-c]pyrrole-(1RS,4aRS,7aRS) sous la forme de cristaux beiges fondant à 126°C.

EXEMPLE 10

A une solution refroidie à 0°C environ de 1,43 g de (-)-diphényl-4,4 oxyde-1 perhydrothiopyrano[2,3-c]pyrrole-(1R*,4aR*,7aR*), et de 0,83 g d'acide (méthoxy-2 phényl)-2 propionique-(S) dans 100 $cm^3$ de dichlorométhane sec, on ajoute 0,06 g d'hydrate d'hydroxybenzotriazole et 1,01 g de chlorhydrate de (diméthylamino-3 propyl)-1 éthyl-3 carbodiimide. Après 2 heures d'agitation à 0°C et 2 heures à 20°C, le mélange réactionnel est lavé par 50 $cm^3$ d'eau puis séché sur sulfate de magnésium, et concentré à sec sous pression réduite (2,7 kPa). Le résidu est cristallisé dans de l'acétonitrile, les cristaux sont essorés, lavés plusieurs fois par de l'éther éthylique, puis séchés.

On obtient 1,56 g de [(-)-(méthoxy-2 phényl)-2 propionyl-(S) ] -6 diphényl-4,4 oxyde-1 erhydrothiopyrano[2,3-c]pyrrole-(1R*, 4aR*, 7aR*) sous la forme de cristaux blancs fondant à 170°C. $[\alpha]^{20}D$ = -316° (c = 0,50; acide acétique).

EXEMPLE 11

En opérant comme décrit précédemment dans l'exemple 1, à partir de 0,49 g d'acide diméthylamino-2 phénylacétique, de 0,50 g de N,N′-carbonyldiimidazole, de 0,70 $cm^3$de triéthylamine et de 1,0 g de chlorhydrate de diphényl-4,4 perhydrothiopyrano[2,3-c]pyrroledioxyde-1,1-(4aRS,7aRS), on obtient 0,55 g de [(diméthylamino-2 phényl)-6 diphényl-4,4 perhydrothiopyrano[2,3-c]pyrrole dioxyde-1,1-(4aRS,7aRS), sous la forme de cristaux blancs fondant à 226°C.

EXEMPLE 12

A une suspension refroidie à 0°C de 0,46 g de chlorhydrate de diphényl-4,4 perhydrothiopyrano[2,3-c]pyrrole dioxyde-1,1-(4aRS,7aRS) dans 10 $cm^3$ de dichlorométhane, on ajoute 0,35 $cm^3$ de triéthylamine, et une solution de 0,17 $cm^3$ de chlorure de phénylacétyle dans 5 $cm^3$ de dichlorométhane. Après 1 heure d'agitation à 0°C puis 1 heure à 20°C, le mélange réactionnel est dilué avec 10 $cm^3$ de dichlorométhane, lavé 2 fois par 30 $cm^3$ d'eau distillée, séché sur sulfate de magnésium et concentré à sec sous pression réduite (2,7 kPa). L'huile obtenue est cristallisée dans 30 $cm^3$ d'éther éthylique, les cristaux sont essorés, et séchés. On obtient 0,50 g de diphényl-4,4 phénylacétyl-6 perhydrothiopyrano[2,3-c]pyrrole dioxyde-1,1-(4aRS,7aRS), sous la forme de cristaux blancs.

Spectre infra-rouge (bandes caractéristiques, $cm^{-1}$): 3050, 3025, 2970, 2930, 1880, 1630, 1595, 1495, 1455, 1425, 1330, 1305, 1140, 1120, 765, 755, 700, 510.

Spectre RMN du proton (DMSO-$d_6$ + $CF_3CCOD$, signaux principaux, à température ambiante on observe le mélange des deux rotamères): 2,48 (mt, 1H, $CH_2$ en 3); 2,8 (mt, 1H, 1H en 5); 3,39 et 3,65 (s et ab J=14, 2H, N-CO-$CH_2$); 6,9 à 7,6 (mt, 15H, aromatiques).

Le chlorohydrate de diphényl-4,4 perhydrothiopyrano[2,3-c]pyrrole dioxyde-1,1-(4aRS,7aRS) peut être préparé de la façon suivante:

On traite 0,58 g de diphényl-4,4 vinyloxycarbonyl-6 perhydrothiopyrano[2,3-c]pyrrole dioxyde-1,1-(4aRS,7aRS) par 25 $cm^3$ d'une solution 5,7 N d'acide chlohydrique dans le dioxane sec pendant 30 minutes en tiédissant. La solution réactionnelle est concentrée à sec sous pression réduite (2,7 kPa) à 50°C. Le résidu est repris par 15 $cm^3$ d'éthanol, la solution obtenue est portée au reflux pendant 30 minutes, puis est concentrée à sec. Le solide obtenu est lavé par de l'éther éthylique, essoré, et séché. On obtient 0,46 g de chlorhydrate de diphényl-4,4 perhydrothiopyrano[2,3-c] dioxyde-1,1-(4aRS,7aRS), sous la forme d'un solide blanc.

Spectre infra-rouge (bandes caractéristiques, $cm^{-1}$): 3055, 3030, 2970, 2935, 2825, 2300, 1600, 1495, 1462, 1335, 1315, 1300, 1140, 1120, 770, 760, 710, 595, 505.

Spectre RMN du proton (DMSO-$d_6$ + $CF_3CCOD$, signaux principaux): 3,84 (ab, 2H, $CH_2$ en 7); 4,0 (mt, 1H, H en 4a); 4,27 (mt, 1H, H en 7a); 7,1 à 7,6 (mt, 10H, aromatiques).

Le diphényl-4,4 vinyloxycarbonyl-6 perhydrothiothiopyrano[2,3-c] pyrrole dioxyde-1,1-(4aRS,7aRS) peut être préparé de la façon suivante:

A une solution de 0,7 g benzyl-6 diphényl-4,4 perhydrothiopyrano[2,3-c]pyrrole dioxyde-1,1-(4aRS,7aRS) dans 10 $cm^3$ de dichloro-1,2 éthane on ajoute 0,16 $cm^3$ de chloroformiate de vinyle. Le mélange est porté au reflux pendant 2 heures puis concentré à sec sous pression réduite (2,7 kPa), à 50°C. Le solide cristallin est lavé par de l'éther éthylique, essoré puis séché. On obtient 0,58 g de diphényl-4,4 vinyloxycarbonyl-6 perhydrothiopyrano[2,3-c]pyrrole dioxyde-1,1-(4aRS,7aRS), sous la forme de cristaux blancs.

Spectre infra-rouge (bandes caractéristiques, $cm^{-1}$): 3080, 3055, 3025, 2990, 2970, 2925, 2885, 1715, 1645, 1595, 1580, 1495, 1415, 1330, 1300, 1150, 1140, 1125, 945, 865, 755, 700, 510.

Spectre RMN du proton (DMSO-$d_6$ + $CF_3COOD$, signaux principaux): 2,5 à 3,45 (mt, 6H, $CH_2$ en 5, en 2 et en 3); 3,8 à 4,2 (mt, 4H, $CH_2$ en 7, H en 4a et H en 7a); 4,46 et 4,72 (2d large, J=6 et J=14, 2x1H, CH=$\underline{CH_2}$); 7,0 (dd, J=14 et 6, 1H, O$\underline{CH}$=-); 7,1 à 7,6 (mt, 10H, aromatiques).

Le benzyl-6 diphényl-4,4 perhydrothiopyrano[2,3-c]pyrrole dioxyde-1,1-(4aRS,7aRS) peut être préparé de la façon suivante:

A une solution de 1,3 g de dihydro-3,4 diphényl-4,4 dioxyde-1,1 2H-thiapyranne et de 1,75 $cm^3$ de N-butoxyméthyl N-triméthylsilylméthyl benzylamine dans 12 $cm^3$ de dichlorométhane anhydre, on ajoute 2 gouttes d'acide trifluoroacétique et agite pendant 30 minutes à 30°C. 1,75 $cm^3$de N-butoxyméthyl N-triméthylsilylméthyl benzylamine et 2 gouttes d'acide trifluoroacétique sont à nouveau ajoutés et le mélange est agité pendant 2 heures à 35°C. Cette dernière opération est répétée une nouvelle fois, et après 1 heure d'agitation, on ajoute 1 g de carbonate de potassium. La suspension est filtrée, le filtrat concentré à sec sous pression réduite (2,7 kPa). Le résidu est chromatographié sur colonne de gel de silice (granulométrie 0,04-0,06 mm , diamètre 3,2 cm, hauteur 35 cm) , en éluant sous une pression de 0,5 bar d'azote par un mélange de cyclohexane et d'acétate d'éthyle (80/20 en volumes) et en recueillant des fractions de 30 $cm^3$. Les fractions 20 à 28 sont réunies et concentrées à sec sous pression réduite (2,7 kPa). On obtient 0,7 g de benzyl-6 diphényl-4,4 perhydrothiopyrano [2,3-c]pyrrole dioxyde-1,1-(4aRS,7aRS), sous la forme de cristaux blancs fondant à 186°C.

Le dihydro-3,4 diphényl-4,4 dioxyde-1,1 2H-thiapyranne peut être préparé de la façon suivante:

A une solution de 1,47 g de dihydro-3,4 diphényl-4,4 oxyde-1 2H-thiapyranne dans 15 $cm^3$ de dichlorométhane sec, on ajoute une solution de 1,12 g d'acide chloro-3 peroxybenzoïque (à 85%) dans 25 $cm^3$de dichlorométhane sec. Après 20 heures d'agitation à 20°C, le mélange réactionnel est lavé par 50 $cm^3$ d'une solution aqueuse à 10% de thiosulfate de sodium puis par 50 $cm^3$ de solution aqueuse saturée d'hydrogénocarbonate de sodium. La phase organique est séchée sur sulfate de magnésium, concentrée à sec sous pression réduite (2,7 kPa).Le résidu cristallin est lavé par de l'ether éthylique, essoré, séché sous pression réduite (2,7 kPa). On obtient 1,3 g de dihydro-3,4 diphényl-4,4 dioxyde-1,1 2H thiapyranne sous la forme de cristaux blancs fondant à 166°C.

Le dihydro-3,4 diphényl-4,4 oxyde-1 2H-thiapyranne peut être préparé de la façon suivante:

En opérant comme ci-dessus, à partir de 2,05 g de dihydro-3,4 diphényl-4,4 2H-thiapyranne et de 1,67 g d'acide chloro-3 peroxybenzoïque (à 85%), on obtient 1,9 g de dihydro-3,4 diphényl-4,4 oxyde-1 2H-thiapyranne, sous la forme d'un solide blanc fondant à 130°C.

Le dihydro-3,4 diphényl-4,4 2H-thiapyranne peut être préparé de la façon suivante:

A une suspension de 2,7 g de diphényl-4,4 oxyde-1 tétrahydrothiapyranne dans 30 $cm^3$ de toluène anhydre, on ajoute 3,95 $cm^3$ d'anhydride acétique. Le mélange est porté au reflux pendant 20 heures et concentré à sec à 60°C sous pression réduite (2,7 kPa puis 0,13 kPa). Le résidu huileux est cristallisé dans de l'oxyde de diisopropyle, les cristaux sont essorés et séchés. On obtient 2,1 g de dihydro-3,4 diphényl-4,4 2H-thiapyranne sous la forme de cristaux blancs, fondant à 78°C.

Le diphényl-4,4 oxyde-1 tétrahydrothiapyranne peut être préparé de la façon suivante:

A une solution refroidie à 0°C de 25,4 g de diphényl-4,4 tétrahydrothiapyranne dans 130 $cm^3$ de dichlorométhane on ajoute en 40 minutes une solution de 20,3 g d'acide chloro-3 peroxybenzoïque (à 85%) dans 300 $cm^3$ de dichlorométhane. Après 2 heures d'agitation à 0°C, on ajoute au mélange 250 $cm^3$ d'une solution aqueuse d'hydrogénocarbonate de potassium à 5% puis agite pendant 15 minutes. La phase organique est lavée à nouveau par 250 $cm^3$ de la solution d'hydrogénocarbonate de potassium, puis est séchée sur sulfate de magnésium, concentrée à sec (après contrôle de l'absence de peroxydes), sous pression réduite (2, 7 kPa). On obtient 26, 9 g de diphényl-4,4 oxyde-1 tétrahydrothiapyranne, sous la forme d'un solide blanc fondant à 122°C.

Le diphényl-4,4 tétrahydrothiapyranne peut être préparé de la façon suivante:

A une suspension de 140,8 g de diphényl-3,3 bis méthanesulfonyloxy-1,5 pentane dans 1400 $cm^3$ de butanol-1 on ajoute 100 g de sulfure de sodium nonahydraté. Le mélange est chauffé au reflux pendant 2 heures puis refroidi à 20°C environ, additionné ensuite de 1000 $cm^3$ d'eau, de 500 $cm^3$ d'acétate d'éthyle, de 500 $cm^3$ de dichlorométhane. Après agitation, la phase organique est séparée, lavée successivement par 1000 $cm^3$ d'eau, 500 $cm^3$ d'acide chlorhydrique 1N, 500 $cm^3$ de solution aqueuse saturée d'hydrogénocarbonate de sodium, 1000 $cm^3$ d'eau, puis séchée sur sulfate de magnésium et concentrée à sec à 60°C sous pression réduite (2,7 kPa). Le résidu est cristallisé dans de l'acétate d'éthyle, les cristaux sont lavés par de l'oxyde de diisopropyle, essorés, et séchés. On obtient 76 g de diphényl-4,4 tétrahydrothiapyranne sous la forme de cristaux blancs fondant à 134°C.

Le diphényl-3,3 bis méthanesulfonyloxy-1,5 pentane peut être préparé de la façon suivante:

A une solution refroidie à -20°C de 95 g de diphényl-3,3 pentane diol-1,5 (préparé selon P. EILBRACHT et coll. Chem. Ber. 118, 825-839 (1985)) dans 950 $cm^3$ de dichlorométhane et de 113 $cm^3$ de triéthylamine, on ajoute en 10 minutes une solution de 62 $cm^3$ de chlorure de méthanesulfonyle dans 100 $cm^3$ de dichlorométhane. Après 2 heures d'agitation à 20°C, le mélange réactionnel est lavé par 2 fois 500 $cm^3$ d'eau, la phase organique est séchée sur sulfate de magnésium, concentrée à sec sous pression réduite (2,7 kPa). Le résidu huileux est cristallisé dans l'éther éthylique, les cristaux sont lavés par de l'éther éthylique, essorés et séchés. On obtient 140 g de diphényl-3,3 bis méthanesulfonyloxy-1,5 pentane sous la forme de cristaux blancs fondant à 99°C.

EXEMPLE 13

A une suspension de 0,12 g d'hydrure de sodium dans 10 $cm^3$ de toluène anhydre, on ajoute en 30 minutes une solution de 1,4 g de diphényl-4,4 [(hydroxy-2 phényl)-2 acétyl]-6 perhydrothiopyrano [2,3-c]pyrrole-(4aRS,7aRS), dans un mélange de diméthylformamide de toluène et de tétrahydrofurane anhydre (15/10/5,5 en volumes). A la solution obtenue, portée à 35°C, on ajoute une solution de 3 g de N-(chloro-3 propyl) N,N diméthylamine dans 10 $cm^3$ de toluène anhydre. Le mélange réactionnel est chauffé au reflux pendant 15 minutes, agité à 20°C pendant 20 heures, dilué ensuite avec 100 $cm^3$ d'acétate d'éthyle, puis lavé 2 fois par 150 $cm^3$ d'eau distillée et 100 $cm^3$ de saumure. La phase organique est séchée sur sulfate de magnésium, concentrée à sec sous pression réduite (2,7 kPa). Le résidu est cristallisé dans de l'oxyde de diisopropyle, les cristaux sont essorés, séchés sous pression réduite (2,7 kPa). On obtient 1,18 g de {[(diméthylamino-3 propoxy)-2 phényl]-2 acétyl}-6 diphényl-4,4 perhydrothiopyrano[2,3-c]pyrrole-(4aRS,7aRS), sous la forme de cristaux beiges, fondant à 130°C.

EXEMPLE 14

A une suspension de 1,5 g de chlorhydrate de diphényl-4,4 perhydrothiopyrano[2,3-c]pyrroledioxyde-1,1-(4aRS,7aRS) et de 0,95 g de chlorhydrate d'éthoxy-1 imino-1 (méthoxy-2 phényl)-2 éthyle dans 15 $cm^3$de dichloro-1,2 éthane, on ajoute goutte à goutte 1,16 $cm^3$ de triéthylamine. Après 20 heures d'agitation à 20°C, le mélange est additionné de 30 $cm^3$ de dichlorométhane puis lavé successivement par 100 $cm^3$ d'eau et 100 $cm^3$ de solution aqueuse de carbonate de potassium à 5%. La phase organique est séchée sur sulfate de magnésium, concentrée à sec sous pression réduite (2,7 kPa). Le résidu est cristallisé dans un mélange d'acétonitrile et d'oxyde de diisopropyle. Les cristaux sont lavés par de l'acétonitrile puis par de l'oxyde de diisopropyle, essorés, et séchés. On obtient 0,83 g d [imino-1 (méthoxy-2 phényl)-2 éthyl-6 diphényl-4,4 perhydrothiopyrano[2,3-c]pyrrole dioxyde-1,1-(4aRS,7aRS) sous la forme de cristaux blancs fondant à 240°C.

EXEMPLE 15

A une solution agitée et refroidie à 0°C de 1,88 g de [(diméthylamino-2 phényl)acétyl]-6 diphényl-4,4 perhydrothiopyrano[2,3-c]pyrrole-(4aRS,7aRS) dans 30 $cm^3$ de dichlorométhane, on ajoute une solution de 0,88 g d'acide chloro-3 peroxybenzoïque (à 85%) dans 15 $cm^3$ de dichlorométhane. Le mélange réactionnel est agité pendant 2 heures à 0°C, lavé 2 fois par 30 $cm^3$ d'une solution aqueuse saturée de bicarbonate de sodium puis par 30 $cm^3$ d'eau. La phase organique est séchée sur sulfate de magnésium, filtrée et concentrée à sec sous pression réduite (2,7 kPa). L'huile jaune obtenue est chromatographiée sur une colonne de gel de silice (0,04 mm-0,06 mm, diamètre 2 cm, hauteur 20 cm), en éluant sous une pression d'azote de 0,6 bar, par un mélange d'acétate d'éthyle et de méthanol (98/2 en volumes), en recueillant des fractions de 60 $cm^3$. Les fractions 9 à 32 sont réunies et concentrées à sec sous pression réduite (2,7 kPa), le résidu est cristallisé dans 10 $cm^3$ d'acétonitrile, les cristaux sont essorés, filtrés et séchés. On obtient 0,93 g de [(diméthylamino-2 phényl)acétyl]-6 diphényl-4,4 oxyde-1 perhydrothiopyrano[2,3-c] pyrrole-(1RS,4aSR,7aSR), sous la forme de cristaux blancs fondant à 204°C.

Les fractions 33 à 50 sont réunies et concentrées à sec puis le résidu est purifié par chromatographie dans les mêmes conditions que précédemment (éluant acétate d'éthyle et méthanol 94/6 en volumes). Les fractions 76 à 86 sont réunies et concentrées à sec sous pression réduite (2,7 kPa. Le résidu est cristallisé dans l'acétonitrile, les cristaux sont éssorés et séchés. On obtient 0,28 g de [(diméthylamino-2 phényl)acétyl]-6 diphényl-4,4 oxyde-1 perhydrothiopyrano[2,3-c]pyrrole-(1RS, 4aRS,7aRS), sous la forme d'un solide blanc fondant à 190°C.

Le [(diméthylamino-2 phényl)acétyl]-6 diphényl-4,4 oxyde-1 perhydrothiopyrano[2,3-c]pyrrole-(1RS, 4aRS,7aRS) peut être aussi préparé de la façon suivante:

On agite pendant 48 heures à 20°C 2,46 g de [(diméthylamino-2 phényl)acétyl]-6 diphényl-4,4 oxyde-1 perhydrothiopyrano[2,3-c] pyrrole-(1RS,4aSR,7aSR) dans 25 $cm^3$ d'un mélange d'acide chlorhydrique concentré (37% d'acide chlorhydrique) et de dioxane (1/2 en volumes). La solution est ensuite neutralisée à 20°C par 20 $cm^3$d'une solution aqueuse d'hydroxyde de sodium 4N. La phase organique décantée est lavée par 50 $cm^3$ d'eau distillée, séchée sur sulfate de magnésium et concentrée à sec sous pression réduite (2,7 kPa) à 30°C. Le résidu huileux est chromatographié sur une colonne de gel de silice (0,04 - 0,06 mm, diamètre 3,6 cm, hauteur 34 cm), en éluant sous une pression d'azote de 0,8 bar, par un mélange d'acétate d'éthyle et de méthanol (95/5 en volumes), en recueillant des fractions de 25 $cm^3$. Les fractions 72 à 92 sont réunies et concentrées à sec sous pression réduite (2,7 kPa) à 40°C. Le résidu est cristallisé dans l'acétate d'éthyle, les cristaux sont lavés par de l'oxyde de diisopropyle, puis essorés et séchés. On obtient 1,08 g de [(diméthylamino-2 phényl)acétyl]-6 diphényl-4,4 oxyde-1 perhydrothiopyrano[2,3-c]pyrrole-(1RS,4aRS,7aRS), sous la forme de cristaux blancs fondant à 190°C.

EXEMPLE 16

A une solution refroidie à 0°C de 1,85 g de {[(pyrrolidinyl-1)-2 phényl] acétyl-6 diphényl-4, 4 perhydrothiopyrano[2, 3-c]pyrrole-(4aRS, 7aRS) dans 30 $cm^3$ de dichlorométhane, on ajoute une solution de 0,83 g d'acide chloro-3 peroxybenzoïque (à 85%) dans 15 $cm^3$ de dichlorométhane. Après 1 heure d'agitation à 0°C et 2 heures à température ambiante, le mélange réactionnel est lavé par 50 $cm^3$ d'une solution aqueuse saturée de bicarbonate de sodium puis par 2 fois 30 $cm^3$ d'eau. La phase organique est séchée sur sulfate de magnésium, concentrée à sec sous pression réduite (2,7 kPa). Le résidu est repris par 19 $cm^3$ d'un mélange d'acide chlorhydrique concentré et de dioxiane (1 /2 en volumes. Après 48 heures d'agitation à 20°C, la solution résultante est additionnée de 15 $cm^3$ de soude 4N, puis extraite avec 30 $cm^3$ de dichlorométhane. La phase organique est lavée par 40 $cm^3$ d'eau puis séchée sur sulfate de magnésium et concentrée à sec. Le résidu est chromatographié sur une colonne de gel de silice (0,04-0,06 mm, diamètre 3,6 cm, hauteur 40 cm) en éluant sous une pression d'azote de 0,8 bar, par mélange d'acétate d'éthyle et de méthanol (95/5 en volumes), en recueillant des fractions de 25 $cm^3$. Les fractions 100 à 119 sont réunies et concentrées à sec sous pression réduite (2,7 kPa). Le résidu est cristallisé dans dans l'acétate d'éthyle, les cristaux sont essorés, séchés. On obtient 0,11 g de {[(pyrrolidinyl-1)-2 phényl]acétyl}-6 diphényl-4,4 oxyde-1 perhydrothiopyrano[2,3-c]pyrrole-(1RS,4aSR,7aSR) sous la forme d'un solide blanc fondant à 190 °C.

EXEMPLE 17

En opérant comme à l'exemple 16, en réunissant et en concentrant à sec les fractions chromatographiques 123 à 148, on obtient un résidu que l'on cristallise dans de l'acétate d'éthyle. Les cristaux sont essorés, lavés par de l'acétate d'éthyle et de l'oxyde de diisopropyle puis séchés. On obtient 0,39 g de {[(pyrrolidinyl-1)-2 phényl]acétyl}-6 diphényl-4,4 oxyde-1 perhydrothiopyrano[2,3-c] pyrrole-(1RS,4aRS,7aRS) sous la forme d'un solide blanc fondant à 198°C.

EXEMPLE 18

A une solution de 0,5 g de (diméthylamino-3 propoxy)-2 phényl]-2 acétyl}-6 diphényl-4,4 perhydrothiopyrano[2,3-c]pyrrole-(4aRS,7aRS) dans 5 $cm^3$ de dichlorométhane sec, on ajoute 0,15 $cm^3$ d'acide trifluoroacétique puis refroidit à 0°C. Après 30 minutes d'agitation à 0°C, on ajoute goutte à goutte une solution d'acide chloro-3 peroxybenzoïque (à 85%) dans 10 $cm^3$ de dichlorométhane. Le mélange réactionnel est agité pendant 2 heures à 0°C, élué ensuite avec 10 $cm^3$de dichlorométhane puis lavé par 20 $cm^3$ de soude 1N et 20 $cm^3$ de solution aqueuse saturée de chlorure de sodium. La phase organique est séchée sur sulfate de magnésium, concentré à sec sous pression réduite (2,7 kPa). Le résidu est chromatographié sur une colonne de gel de silice (granulométrie 0,04-0,06 mm, diamètre 2 cm, hauteur 30 cm) en éluant sous une pression de 1 bar d'azote par un mélange d'acétate d'éthyle, d'acide acétique et d'eau (80/10/10) et en recueillant des fractions de 25 $cm^3$. Les fractions 13 à 21 sont réunies et concentrées à sec sous pression réduite (2,7 kPa). Le résidu est cristallisé dans un mélange d'acétate d'éthyle et d'oxyde de diisopropyle, les cristaux sont essorés puis séchés. On obtient 0,13 g de {[(diméthylamino-3 propoxy)-2 phényl]-2 acétyl}-6 diphényl-4,4 perhydrothiopyrano[2,3-c]pyrrole-(1RS,4aSR,7aSR) sous la forme d'un solide beige fondant à 146°C.

EXEMPLE 19

En opérant comme décrit précédemment dans l'exemple 15, à partir de 0,99 g de diphényl-4,4 phénylacétyl-6 perhydrothiopyrano[2,3-c] pyrrole-(4aRS,7aRS) et de 0,49 g d'acide chloro-3 peroxybenzoïque, on obtient après chromatographie 0,54 g de diphényl-4,4 phénylacétyl-6 oxyde-1 perhydrothiopyrano[2,3-c]pyrrole-(1RS,4aSR,7aSR) sous la forme de cristaux blancs fondant à 217°C.

EXEMPLE 20

En opérant comme décrit précédemment dans l'exemple 15, à partir de 0,99 g de diphényl 4,4 phénylacétyl-6 perhydrothiopyrano [2,3-c]pyrrole-(4aRS,7aRS) et de 0,49 g d'acide chloro-3 peroxybenzoïque, on obtient après purification chromatographique, 0,17 g de diphényl-4,4 phénylacétyl-6 oxyde-1 perhydrothiopyrano[2,3-c]pyrrole-(1RS,4aRS,7aRS), sous la forme de cristaux blancs fondant à 226°C.

EXEMPLE 21

En opérant comme précédemment à l'exemple 15, à partir de 0,95 g de diphényl-4,4 [(méthoxy-2 phényl)acétyl]-6 perhydrothiopyrano[2,3-c] pyrrole-(4aRS,7aRS) et de 0,43 g d'acide chloro-3 peroxybenzoïque (à 85%), on obtient après purification chromatographique 1,0 g de [(méthoxy-2 phényl)acétyl]-6 diphényl-4,4 oxyde-1 perhydrothiopyrano [2,3-c]pyrrole(1RS,4aSR,7aSR), sous la forme d'un solide blanc fondant à 240°C, et 0,15 g de [(méthoxy-2 phényl)acétyl]-6 diphényl-4,4 oxyde-1 perhydrothiopyrano[2,3-c]pyrrole-(1RS,4aRS, 7aRS), sous la forme d'un solide blanc fondant à 254°C.

EXEMPLE 22

A une solution refroidie à 0°C de 1,46 g de [(méthoxy-2 phényl)-2 propionyl-(S)}-6 diphényl-4,4 perhydrothiopyrano[2,3-c]pyrrole mélange des formes (4aR,7aR) et 4aS,7aS) dans 15 $cm^3$ de dichlorométhane, on

ajoute une solution de 0,69 g d'acide chloro-3 peroxybenzoique (à 85%) dans 15 $cm^3$ de dichlorométhane. Après agitation 3 heures à +5°C, le mélange réactionnel est lavé par 30 $cm^3$ de solution aqueuse saturée de bicarbonate de sodium et par 2 fois 30 $cm^3$ d'eau, puis séché sur sulfate de magnésium et concentré à sec sous pression réduite (2,7 kPa). Le résidu est traité pendant 48 heures à 20°C par un mélange d'acide chlorhydrique concentré et de dioxanne (1/2 en volumes). La solution résultante est lavée par 12 $cm^3$ de solution aqueuse d'hydroxyde de sodium 4N, par 20 $cm^3$ d'eau, puis est concentrée à sec à 40°C sous pression réduite. Le résidu est chromatographié sur colonne de gel de silice (granulométrie 0,02-0,045 mm , diamètre 3,6 cm, hauteur 40 cm), en éluant sous une pression de 0,8 bar d'azote par un mélange d'acétate d'éthyle et de méthanol (95/5 en volumes) et en recueillant des fractions de 60 $cm^3$. Les fractions 22 à 42 sont réunies et concentrées à sec sous pression réduite (2,7 kPa). On obtient 0,4 g de [(méthoxy-2 phényl)-2 propionyl-(S)]-6 diphényl-4,4 oxyde-1 perhydrothiopyrano[2,3-c] pyrrole mélange des formes (1R,4aR,7aR) et (1S,4aS,7aS) sous la forme d'un solide blanc.

Spectre infra-rouge (bandes caractéristiques, $cm^{-1}$): 3060, 3025, 2970, 2935, 2880, 2840, 1640, 1595, 1490, 1460, 1445, 1425, 1245, 1020, 755, 705.

Spectre RMN du proton (à température ambiante, on observe le mélange de deux rotamères de chaque forme signaux caractéristiques): 1,2-1,4 (mt, 3H, $CH_3$); 3,41, 3,60 et 3,87 (3s, 3H, $OCH_3$); 6,5 à 7,4 (mt, 14H aromatiques).

EXEMPLE 23

En opérant comme à l'exemple 22, en réunissant et en évaporant à sec les fractions chromatographiques 13 à 20, on obtient un résidu que l'on cristallise dans de l'acétonitrile. Les cristaux sont essorés, lavés par de l'acétonitrile et de l'oxyde de diisopropyle puis séchés. On obtient 0,02 g de [(méthoxy-2 phényl)-2 propionyl-(S)]-6 diphényl-4,4 oxyde-1 perhydrothiopyrano[2,3-c]pyrrole-mélange des formes (1R,4aS,7aS) et (1S,4aR,7aR) sous la forme d'un solide blanc.

Spectre infra-rouge (bandes caractéristiques, $cm^{-1}$): 3060, 3030, 3000, 2970, 2935, 2880, 2840, 1640, 1595, 1490, 1485, 1460, 1445, 1420, 1370, 1240, 1050, 1040, 1030, 755, 705.

Spectre RMN du proton (DMSO-$d_6$ + $CF_3COOD$, à température ambiante, on observe le mélange des deux rotamères ; signaux caractéristiques): 1,1 - 1,3 (mt,3H, $CH_3$); 3,35 et 3,81 (2s, 3H, $OCH_3$); 6,7 à 7,5 (mt, 14H aromatiques).

EXEMPLE 24

A une solution, placée sous atmosphère d'azote, de 0,3 $cm^3$ de tétraisopropylate de titane dans 10 $cm^3$ de dichlorométhane et 0,018 $cm^3$ d'eau, on ajoute une solution de 0,443 g de [(méthoxy-2 phényl)acétyl]-6 diphényl-4,4 perhydrothiopyrano[2,3-c]pyrrole-(4aRS,7aRS) dans 4,0 $cm^3$ de dichlorométhane. Le mélange est refroidi à -20°C, puis additionné de 0,16 $cm^3$ d'une solution aqueuse à 70% de tertiobutylhydroperoxyde. Après 6 heures d'agitation à -20°C, la suspension blanche obtenue est additionnée de 0,01 $cm^3$ de la solution de tertiobutylhydroperoxyde. Après 1 heure 30 minutes à -20°C, on ajoute au mélange réactionnel 2,0 $cm^3$ d'eau, filtre le gel obtenu. La phase dichlorométhane est séchée sur sulfate de sodium, concentrée à sec sous pression réduite (2,7 kPa). Le résidu est cristallisé dans de l'acétonitrile, les cristaux sont repris par du dichlorométhane et la solution obtenue est chromatographiée sur colonne de gel de silice (granulométrie 0,04-0,06 mm, diamètre 2 cm, hauteur 20 cm), en éluant sous une pression de 0,6 bar d'azote par un de l'acétate d'éthyle puis par un mélange d'acétate d'éthyle et de méthanol (90/10 en volumes) et en recueillant des fractions de 30 $cm^3$. Les fractions 5 et 6 sont réunies et concentrées à sec sous pression réduite (2,7 kPa). On obtient 0,06 g de [(méthoxy-2 phényl)acétyl]-6 diphényl-4,4 dioxyde-1,1 perhydrothiopyrano[2,3-c] pyrrole-(4aRS,7aRS) sous la forme d'un solide blanc fondant à 258°C. Les fractions 24 et 25 sont réunies et concentrées à sec. On obtient, après cristallisation dans dans l'acétonitrile 0,26 g de [(méthoxy-2 phényl)acétyl]-6 diphényl -4,4 oxyde-1 perhydrothiopyrano[2,3-c]pyrrole(1RS,4aSR,7aSR) sous la forme d'un solide blanc fondant à 240°C.

La présente invention concerne également les compositions pharmaceutiques constituées par un produit de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en

particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les produits selon l'invention peuvent être particulièrement utiles dans le traitement des douleurs d'origine traumatique, post-chirurgicale, menstruelle, céphaliques, dans les traitements de l'anxiété, des psychoses, de la maladie de Parkinson, de la schizophrénie, de la maladie d'Alzeimer, dans les traitements myorelaxants, dans les traitements des manifestations spasmodiques, douloureuses et inflammatoires des voies digestives (colites ulcéreuses, syndrome du colon irritable, maladie de Crohn), des voies urinaires (cystites) et des voies respiratoires (asthme, rhinites) ou en gynécologie et dans les traitements des migraines. Les nouveaux dérivés de thiopyranopyrrole sont également utiles dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses et dans les troubles inflammatoires occulaires ou dentaires.

Les produits selon l'invention peuvent également trouver une application dans les traitements des troubles cardiovasculaires tels de l'hypotension.

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

Exemple

On prépare, selon la technique habituelle, des comprimés de produit actif ayant la composition suivante :

- {{[(pyrrolidinyl-1)-3 propoxy-2]phényl}acétyl}-6 diphényl-4,4 oxyde-1 perhydrothiopyrano[2,3-c] pyrrole-(1RS,4aRS,7aRS) ........................ 25 mg
- amidon ........................................ 83 mg
- silice ........................................ 30 mg
- stéarate de magnésium ........................ 3 mg

**Revendications**

**1 -** Nouveau dérivé de thiopyranopyrrole caractérisé en ce qu'il répond à la formule générale :

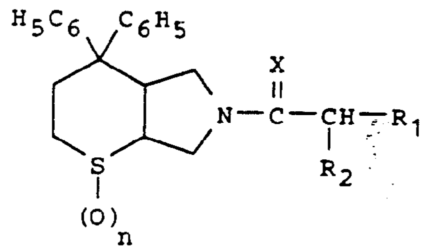

dans laquelle

- le symbole X représente un atome d'oxygène, ou un radical NH,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués (par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy ou hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino, et
- n est un nombre entier de 0 à 2,

les radicaux alcoyle et acyle cités ci-dessus étant droits ou ramifiés et contenant 1 à 4 atomes de carbone, sous ses formes stéréoisomères ou leurs mélanges, ainsi que ses sels lorsqu'ils existent.

**2** - Le {[(diméthylamino-3 propoxy-2) phényl] acétyl)-6 diphényl-4,4-oxyde-1 perhydrothiopyrano[2,3-c]pyrrole sous ses formes stéréoisomères et leurs mélanges.

**3** - Le {[(pyrrolidinyl-1)-3 propoxy-2] phényl} acétyl}-6 diphényl-4,4 oxyde-1 perhydrothiopyrano[2,3-c]pyrrole sous ses formes stéréoisomères et leurs mélanges.

**4** - Le [(méthoxy-2 phényl)-2 propionyl-(S)]-6 diphényl-4,4 oxyde-1 perhydrothiopyrano[2,3-c]pyrrole sous ses formes stéréoisomères et leurs mélanges.

**5** - Le {[(diméthylamino-3 propoxy)-2 phényl]-2 acétyl}-6 diphényl-4,4 perhydrothiopyrano[2,3-c]pyrrole sous ses formes stéréoisomères et leurs mélanges.

**6** - Procédé de préparation d'un nouveau dérivé de thiopyranopyrrole selon la revendication 1, caractérisé en ce que l'on fait agir un acide ou un dérivé réactif de l'acide de formule générale :

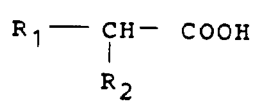

dans laquelle $R_1$ et $R_2$ sont définis comme dans la revendication 1, sur un dérivé de thiopyranopyrrole de formule générale :

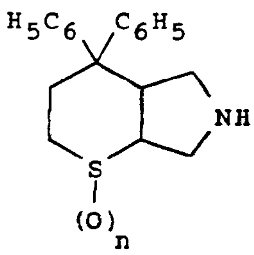

dans laquelle le symbole n est défini comme dans la revendication 1, puis transforme éventuellement l'amide obtenu en une amidine pour laquelle X représente un radical NH et transforme éventuellement le produit obtenu en un sel lorsqu'ils existent.

**7 -** Procédé de préparation d'un dérivé de thiopyranopyrrole selon la revendication 1, pour lequel le symbole $R_1$ représente un radical alcoyloxyphényle dont la partie alcoyle est éventuellement substituée, le symbole $R_2$ est autre qu'un radical hydroxy et les symboles n et X sont définis comme dans la revendication 1, caractérisé en ce que l'on fait agir un dérivé halogéné de formule générale :

$$R_4\text{-Hal}$$

dans laquelle $R_4$ est un radical alcoyle, éventuellement substitué [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées un hétérocycle tel que défini précédemment dans la revendication 1] et Hal est un atome d'halogène, sur un dérivé de thiopyranopyrrole selon la revendication 1, pour lequel $R_1$ est un radical hydroxphényle, puis élimine le cas échéant le radical protecteur d'hydroxy et transforme éventuellement le produit obtenu en un sel lorsqu'ils existent.

**8 -** Procédé de préparation d'un dérivé de thiopyranopyrrole selon la revendication 1 pour lequel X est un radical NH et les symboles $R_1$, $R_2$ et n sont définis comme dans la revendication 1, caractérisé en ce que l'on fait agir un produit de formule générale :

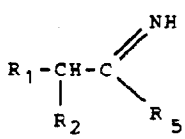

éventuellement à l'état de sel, dans laquelle $R_1$ et $R_2$ sont définis comme dans la revendication 1 et $R_5$ représente un radical alcoyloxy droit ou ramifié contenant 1 à 4 atomes de carbone ou un radical méthylthio, éthylthio, benzylthio ou alcoyloxycarbonylméthylthio, sur un dérivé de thiopyranopyrrole de formule générale :

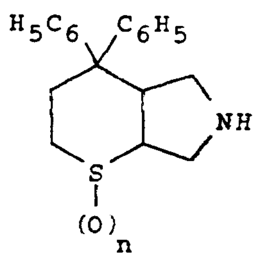

dans laquelle n est défini comme dans la revendication 1, puis, lorsqu'ils existent, transforme éventuellement le produit en un sel.

**9 -** Procédé de préparation d'un dérivé de thiopyranopyrrole selon la revendication 1 pour lequel n égale 1 ou 2, caractérisé en ce que l'on oxyde le dérivé correspondant selon la revendication 1 pour lequel n égale 0, puis transforme éventuellement le produit obtenu en un sel lorsqu'ils existent.

**10 -** Composition pharmaceutique caractérisée en ce qu'elle contient au moins un produit selon l'une des revendications 1 à 5, à l'état pur ou sous forme d'association avec un ou plusieurs adjuvants ou diluants compatibles et pharmaceutiquement acceptables.

Office européen des brevets

# RAPPORT DE RECHERCHE EUROPEENNE

Numero de la demande

EP 92 40 1331

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 058 567 (WARNER-LAMBERT CO.)<br>* abrégé *<br>--- | 1,10 | C07D495/04<br>A61K31/40<br>//(C07D495/04,<br>335:00,209:00) |
| A | EP-A-0 093 805 (WARNER-LAMBERT CO.)<br>* abrégé *<br>--- | 1,10 | |
| A,D | US-A-4 042 707 (W.C. RIPKA)<br>* colonne 31, tableau I *<br>--- | 1 | |
| A,D | EP-A-0 068 822 (ROHM AND HAAS CO.)<br>* abrégé *<br>----- | 1 | |

| DOMAINES TECHNIQUES RECHERCHES (Int. Cl.5 ) |
|---|
| C07D |

Le présent rapport a été établi pour toutes les revendications

| Lieu de la recherche | Date d'achèvement de la recherche | Examinateur |
|---|---|---|
| BERLIN | 21 AOUT 1992 | CHRISTIAN HASS |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)